(19) Europäisches Patentamt
European Patent Office
Office européen des brevets

(11) **EP 3 651 804 B1**

(12) **EUROPÄISCHE PATENTSCHRIFT**

(45) Veröffentlichungstag und Bekanntmachung des Hinweises auf die Patenterteilung:
**23.08.2023  Patentblatt 2023/34**

(21) Anmeldenummer: **18737623.1**

(22) Anmeldetag: **11.07.2018**

(51) Internationale Patentklassifikation (IPC):
*A61K 9/107* (2006.01)    *A61P 37/00* (2006.01)
*A61P 35/00* (2006.01)    *A61P 25/16* (2006.01)
*A61P 29/00* (2006.01)    *A61P 25/28* (2006.01)
*A61K 36/324* (2006.01)    *A61K 31/12* (2006.01)
*A61P 3/00* (2006.01)    *A61P 3/04* (2006.01)
*A61P 3/06* (2006.01)    *A61P 3/10* (2006.01)
*A61K 47/26* (2006.01)    *A61K 36/185* (2006.01)
*A61K 36/9066* (2006.01)

(52) Gemeinsame Patentklassifikation (CPC):
(C-Sets verfügbar)
**A61K 9/0095; A61K 9/1075; A61K 31/12;
A61K 31/352; A61K 36/185; A61K 36/484;
A61K 36/9066; A61K 47/26; A61P 1/16;
A61P 3/04; A61P 3/06; A61P 3/10;** A61K 2236/00

(Forts.)

(86) Internationale Anmeldenummer:
**PCT/EP2018/068729**

(87) Internationale Veröffentlichungsnummer:
**WO 2019/011954 (17.01.2019 Gazette 2019/03)**

(54) **SOLUBILISAT MIT CURCUMIN UND BOSWELLIA UND XANTHOHUMOL**

SOLUBILISATE WITH CURCUMIN, BOSWELLIA, AND XANTHOHUMOL

SOLUBILISAT COMPRENANT DE LA CURCUMINE, DU BOSWELLIA ET DU XANTHOHUMOL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.07.2017   PCT/EP2017/067382
11.07.2017   PCT/EP2017/067381
11.07.2017   DE 102017115496**

(43) Veröffentlichungstag der Anmeldung:
**20.05.2020   Patentblatt 2020/21**

(73) Patentinhaber: **Aquanova AG
64295 Darmstadt (DE)**

(72) Erfinder: **BEHNAM, Dariush
64380 Rossdorf (DE)**

(74) Vertreter: **Tesch, Sabine
Augspurger Tesch Friderichs
Patent- und Rechtsanwälte PartG mbB
Kaiserstraße 39
55116 Mainz (DE)**

(56) Entgegenhaltungen:
**EP-A1- 0 755 940        EP-A1- 1 431 385
WO-A2-2007/006497        DE-A1-102006 024 911
DE-U1-202012 012 130     US-A1- 2016 081 975**

• **REJI KIZHAKKEDATH: "Clinical evaluation of a formulation containing Curcuma longa and Boswellia serrata extracts in the management of knee osteoarthritis", MOLECULAR MEDICINE REPORTS, Bd. 8, Nr. 5, 1. November 2013 (2013-11-01), Seiten 1542-1548, XP055462856, GR ISSN: 1791-2997, DOI: 10.3892/mmr.2013.1661**

- DATABASE EMBASE [Online] ELSEVIER SCIENCE PUBLISHERS, AMSTERDAM, NL; 1. September 2017 (2017-09-01), KHAYYAL M T: "Novel formulations of Curcumin, Boswellia and Xanthohumol extracts markedly enhance their individual and combined anti-inflammatory activity", XP002783955, Database accession no. EMB-621379886 & ZEITSCHRIFT FUR PHYTOTHERAPIE 20170901 HIPPOKRATES VERLAG GMBH NLD, Bd. 38, Nr. Supplement 1, 1. September 2017 (2017-09-01), ISSN: 1438-9584
- ALEXA KOCHER ET AL: "The oral bioavailability of curcuminoids in healthy humans is markedly enhanced by micellar solubilisation but not further improved by simultaneous ingestion of sesamin, ferulic acid, naringenin and xanthohumol", JOURNAL OF FUNCTIONAL FOODS, Bd. 14, 1. April 2015 (2015-04-01), Seiten 183-191, XP055435032, NL ISSN: 1756-4646, DOI: 10.1016/j.jff.2015.01.045
- ZAMZOW DANIEL R ET AL: "Xanthohumol improved cognitive flexibility in young mice", BEHAVIOURAL BRAIN RESEARCH, Bd. 275, 1. September 2014 (2014-09-01), Seiten 1-10, XP029077816, ISSN: 0166-4328, DOI: 10.1016/J.BBR.2014.08.045

(52) Gemeinsame Patentklassifikation (CPC): (Forts.)

C-Sets
A61K 31/12, A61K 2300/00;
A61K 31/352, A61K 2300/00;
A61K 36/185, A61K 2300/00;
A61K 36/484, A61K 2300/00;
A61K 36/9066, A61K 2300/00

**EP 3 651 804 B1**

**Beschreibung**

Die Erfindung betrifft ein Solubilisat mit Curcumin und Boswellia und Xanthohumol gemäß Anspruch 1. Des Weiteren betrifft die Erfindung ein Fluid enthaltend ein derartiges Solubilisat, eine Kapsel gefüllt mit einem solchen Solubilisat beziehungsweise Fluid und ein Nahrungsergänzungsmittel und/oder Arzneimittel enthaltend ein solches Solubilisat.

Curcumin wird als Wirkstoff basierend auf verschiedenen möglichen pharmakologischen Eigenschaften diskutiert. Beispielsweise gibt es Anhaltspunkte für die antioxidative und auch für die antiinflammatorische Wirkung des Curcumins ebenso wie für die Wirksamkeit gegen Viren und Bakterien sowie gegen Krebs. Indikationen könnten daher beispielsweise Parkinson, Alzheimer, Diabetes, kolorektale Tumoren, Bauchspeicheldrüsenkrebs und Leberfunktionsstörungen sein.

Um nach oraler Einnahme in den Blutkreislauf gelangen zu können, muss der Wirkstoff die Dünndarm-Blutschranke passieren, wird dann in der Leber metabolosiert und gelangt als bioverfügbarer Anteil in die Lebervene. Der Rest des insgesamt eingenommenen und im Körper freigesetzten Wirkstoffes wird entweder mikrobiell im Darm abgebaut oder mit den Fäzes beziehungsweise der Galle eliminiert.

Eine Toxizität aufgrund der erfindungsgemäßen Micellierung des Wirkstoffes im Vergleich zur nativen Form konnte anhand von Untersuchungen mit MTT-Assays zur Zelllebensfähigkeit ("viability") ausgeschlossen werden. Der Nachweis der Zellvitalität mittels MTT-Test beruht dabei auf der Reduktion des gelben, wasserlöslichen Farbstoffs 3-(4,5-Dimethylthiazol-2-yl)-2,5-diphenyltetrazoliumbromid (MTT) in ein blau-violettes, wasserunlösliches Formazan.

Das Extrakt aus dem Harz des Weihrauchbaumes, *Boswellia serrata* Extrakt, enthält mehrere pentazyklische Triterpene, welche zusammen häufig als gesamte Boswelliasäuren ("total BAs") bezeichnet werden. Mit dem Begriff "Boswelliasäuren" wird eine Gruppe chemischer Verbindungen bezeichnet, die natürlich in dem genannten Harz der Weihrauchbäume vorkommen. Die beiden Grundstrukturen sind die α-Boswelliasäure und die β-Boswelliasäure. Von den Boswelliasäuren sind auch einige Derivate bekannt, insbesondere Verbindungen, die an Position 11 eine Ketogruppe tragen und/oder an Position 3 acetyliert sind. Derzeit gelten im Hinblick auf pharmakologische Effekte insbesondere die Boswelliasäuren "αBA" α-Boswelliasäure und "βBA" β-Boswelliasäure sowie deren Derivate "KBA" 11-keto-β-Boswelliasäure (CAS 17019-92-0) und "AKBA" 3-O-Acetyl-11-keto-β-Boswelliasäure (CAS 67416-16-9) sowie "AαBA" 3-O-Acetyl- α-Boswelliasäure und "AßBA" 3-O-Acetyl-β-Boswelliasäure als bedeutungsvoll. Besonders dem Derivat AKBA wird eine entzündungshemmende Wirkung zugeschrieben.

Im Rahmen der vorliegenden Anmeldung wird der Ausdruck "Boswellia", insbesondere in dem Begriff "Boswellia-Solubilisat" in dem Sinne gebraucht, dass sich Ausdruck "Boswellia" auf die Wirkstoffe aus dem Harz des Weihrauchbaumes bezieht, also auf zumindest eine Boswelliasäure und/oder zumindest ein Derivat einer Boswelliasäure bezieht. Der Ausdruck "Boswelliasäure-Solubilisat" bezeichnet dabei eine mizellare Formulierung zumindest einer Boswelliasäure. Diese kann auch zumindest ein Boswelliasäurederivat enthalten.

Xanthohumol ist ein natürlich in Hopfen vorkommendes Flavonoid. Dabei handelt sich um ein prenyliertes Pflanzenpolyphenol, das den Chalkonen zugeordnet wird und bisher ausschließlich im Hopfen nachgewiesen werden konnte. Dabei weisen die Bitterhopfensorten einen deutlich höheren Gehalt an Xanthohumol auf als Aromasorten. In Tests zeigte sich Xanthohumol als wirksam gegen die Entstehung und Entwicklung von Krebszellen. In Laborversuchen konnte zudem festgestellt werden, dass Xanthohumol die Nervenzellen des Gehirns schützen kann und dadurch möglicherweise helfen könnte, bei Erkrankungen wie Alzheimer oder Parkinson den Krankheitsverlauf zu verlangsamen.

Beispielsweise wird unter http://www.besserlaengerleben.at/gesund-und-fit/hopfen-hilft-gegen-cholesterin-und-blutzucker.html über Untersuchungen berichtet, nach denen Xanthohumol den Plasmaspiegel bei PCSK9 zu senken scheint, einem Protein, das eine wichtige Rolle bei den Cholesterinwerten spielt. Eine Senkung der PCSK9-Werte könnte den Abbau des LDL-Cholesterins aus dem Blut verbessern. Wissenschaftler der Oregon State University haben nachgewiesen, dass die Einnahme großer Mengen von Xanthohumol bei Versuchstieren zu Verbesserungen beim metabolischen Syndrom und einer verringerten Gewichtszunahme führen kann. Diese Forschungsergebnisse könnten zu neuen Behandlungsansätzen bei Fettleibigkeit, hohen Cholesterinwerten und erhöhtem Blutzucker führen. Die Kombination dieser gesundheitlichen Probleme, bekannt als das metabolische Syndrom, gehören heute mit Herz-Kreislauf-Erkrankungen und Typ-2-Diabetes in den Industrieländern zu den häufigsten Todesursachen.

Xanthohumol kommt natürlich im Hopfen und somit in Bier vor. Die höchsten in der Studie eingesetzten Mengen würden beim Menschen einer Dosis von 350 Milligramm pro Tag für eine Person entsprechen. Dieser Wert übersteigt jedoch deutlich jenen, der durch eine normale Nahrungsaufnahme erreicht werden kann. Eine Einnahme über ein Nahrungsergänzungsmittel wäre jedoch theoretisch problemlos möglich.

Als Nahrungsergänzungsmittel sind derzeit Hopfenextrakte kommerziell erhältlich. Allerdings hat es sich gezeigt, dass die Bioverfügbarkeit des Xanthohumols bei oraler Aufnahme von Hopfenextrakten nur gering ist.

Mit dem Begriff "Wirkstoff" wird im Rahmen dieser Anmeldung ein Stoff bezeichnet, der in pharmazeutisch wirksamer Konzentration vorliegt und vorzugsweise zum Zweck einer pharmazeutischen Wirkung beigegeben wird. Dabei sind unter der Bezeichnung des entsprechenden Wirkstoffes auch solche Stoffe zu verstehen, die im Körper in den jeweiligen Wirkstoff und/oder in seine biologisch aktive Form umgewandelt werden.

US 2016/0081975 A1 offenbart ein nicht-wässriges Prä-Gel-Konzentrat enthaltend Curcumin, Boswellia-Extrakt, Hopfenextrakt, TPGS (D-alpha-Tocopherol-Polyethylenglycol-Succinat), Lecithin und andere Bestandteile.

Der Erfinder stellte sich daher die Aufgabe, eine Formulierung bereitzustellen, welche die gesundheitsfördernden bis heilenden Eigenschaften von Curcumin und Boswellia und Xanthohumol dem menschlichen oder tierischen Organismus zugänglich macht. Insbesondere ist es eine Aufgabe der Erfindung, eine möglichst hoheBioverfügbarkeit von Curcumin und Boswellia und Xanthohumol zu ermöglichen.

Diese Aufgaben werden in überraschend einfacher Weise gelöst mit einem Solubilisat nach Anspruch 1. Dieses enthält Curcumin mit einem Anteil von kleiner oder gleich 10 Gew.-%, bevorzugt kleiner oder gleich 8 Gew.-%, besonders bevorzugt 3 Gew.-% bis 7 Gew.-%, eine oder mehrere Boswelliasäuren und/oder eine oder mehrere Boswelliasäurederivate, welche ausgewählt sind aus der Gruppe, die "KBA" 11-keto-$\beta$-Boswelliasäure (CAS 17019-92-0), "AKBA" 3-O-Acetyl-11-keto-$\beta$-Boswelliasäure (CAS 67416-16-9), "A$\alpha$BA" 3-O-Acetyl-$\alpha$-Boswelliasäure und "A$\beta$BA" 3-O-Acetyl-$\beta$-Boswelliasäure umfasst, mit einem Anteil von insgesamt kleiner oder gleich 10 Gew.-%, bevorzugt kleiner oder gleich 8 Gew.-%, besonders bevorzugt 4,7 Gew.-% bis 6,6 Gew.-%, Xanthohumol mit einem Anteil von kleiner oder gleich 10 Gew.-%, bevorzugt kleiner oder gleich 5 Gew.-%, besonders bevorzugt 1 Gew.-% bis 3 Gew.-%, und zumindest einen Emulgator, nämlich Polysorbat 80 oder Polysorbat 20 oder einer Mischung aus Polysorbat 20 und Polysorbat 80, wobei der Emulgatoranteil, insbesondere der Polysorbatanteil, bei mindestens 70 Gew-%, bevorzugt im Bereich zwischen 75 Gew-% und 95 Gew-%, besonders bevorzugt im Bereich zwischen 79 Gew-% und 88 Gew-% liegt.

In einer bevorzugten Ausführungsform der Erfindung besteht das Solubilisat aus Curcumin mit einem Anteil von kleiner oder gleich 10 Gew.-%, bevorzugt kleiner oder gleich 8 Gew.-%, besonders bevorzugt 3 Gew.-% bis 7 Gew.-%, einer oder mehreren Boswelliasäuren und/oder einer oder mehreren Boswelliasäurederivate, welche ausgewählt sind aus der Gruppe, die "KBA" 11-keto-$\beta$-Boswelliasäure (CAS 17019-92-0), "AKBA" 3-O-Acetyl-11-keto-$\beta$-Boswelliasäure (CAS 67416-16-9), "A$\alpha$BA" 3-O-Acetyl-$\alpha$-Boswelliasäure und "A$\beta$BA" 3-O-Acetyl-$\beta$-Boswelliasäure umfasst, mit einem Anteil von insgesamt kleiner oder gleich 10 Gew.-%, bevorzugt kleiner oder gleich 8 Gew.-%, besonders bevorzugt 4,7 Gew.-% bis 6,6 Gew.-% und Xanthohumol mit einem Anteil von kleiner oder gleich 10 Gew.-%, bevorzugt kleiner oder gleich 5 Gew.-%, besonders bevorzugt 1 Gew.-% bis 3 Gew.-%, sowie zumindest einem Emulgator, nämlich Polysorbat 80 oder Polysorbat 20 oder einer Mischung aus Polysorbat 20 und Polysorbat 80, wobei der Emulgatoranteil, insbesondere der Polysorbatanteil, bei mindestens 70 Gew-%, bevorzugt im Bereich zwischen 75 Gew-% und 95 Gew-%, besonders bevorzugt im Bereich zwischen 79 Gew-% und 88 Gew-% liegt.

Aufgrund des hohen Anteils an Boswellia sieht die Erfindung in einer vorteilhaften Ausgestaltung vor, dass das Solubilisat als Quelle dass das Solubilisat als Quelle für die eine oder mehreren Boswelliasäuren und/oder eine oder mehrere Boswelliasäurederivate ein durch Extraktion mittels Essigsäure-Ethylester aus dem Harz der Pflanze *Boswellia serrata* gewonnenes Extrakt enthält, wobei Boswelliasäuren in diesem Auszug in einer Konzentration von mindestens 85 Gew.-% vorliegen.

Es hat sich herausgestellt, dass je nachdem, wie viel Boswellia solubilisiert werden soll, und insbesondere auch in Abhängigkeit von der Frage, ob weitere Wirkstoffe zusätzlich zu Boswellia micelliert werden sollen, das Massenverhältnis von Emulgator zu Boswelliasäuren und/oder zu Boswelliasäuren und zumindest einem ihrer Derivate im Bereich zwischen 20:1 und 3:1, bevorzugt im Bereich zwischen 16:1 und 4:1, bevorzugt im Bereich zwischen 14:1 bis 5:1 liegt.

Aufgrund des hohen Anteils an Xanthohumol sieht die Erfindung in einer vorteilhaften Ausgestaltung vor, dass das Solubilisat als Quelle für Xanthohumol einen ethanolischen Auszug der Hartharze aus Hopfen enthält, wobei Xanthohumol in diesem Auszug in einer Konzentration im Bereich zwischen 65 Gew.-% und 95 Gew.-%, bevorzugt auf eine Konzentration im Bereich von 80 Gew.-% bis 92 Gew.-% vorliegt. Insbesondere das unten näher erläuterte Produkt "Xantho-Flav pur" kann im Rahmen der Erfindung als Xanthohumolquelle verwendet werden.

Es hat sich herausgestellt, dass je nachdem, wie viel Xanthohumol solubilisiert werden soll, und insbesondere auch in Abhängigkeit von der Frage, ob weitere Wirkstoffe zusätzlich zu Xanthohumol micelliert werden sollen, das Massenverhältnis von Emulgator, insbesondere von Polysorbat 80, zu Xanthohumol im Bereich zwischen 30:1 und 3:1, bevorzugt im Bereich zwischen 25:1 und 5:1, bevorzugt im Bereich zwischen 9,8:1 bis 6,6:1 angepasst werden kann.

Je nachdem, wie viel Curcumin im Solubilisat mit Curcumin und Boswellia und Xanthohumol zusätzlich zu Boswellia und Xanthohumol micelliert vorliegen soll, kann im Rahmen der Erfindung das Verhältnis von Emulgator zu Curcumin im Bereich zwischen 30:1 und 3:1, bevorzugt im Bereich zwischen 25:1 und 9:1, bevorzugt im Bereich zwischen 23:1 bis 12:1 gewählt werden. Entsprechend kann das Verhältnis von Emulgator zu Boswelliasäuren und/oder zu Boswelliasäuren und zumindest einem ihrer Derivate im Bereich zwischen 20:1 und 3:1, bevorzugt im Bereich zwischen 16:1 und 4:1, bevorzugt im Bereich zwischen 14:1 bis 5:1 beziehungsweise das Verhältnis von Emulgator, insbesondere von Polysorbat 80, zu Xanthohumol im Bereich zwischen 30:1 und 3:1, bevorzugt im Bereich zwischen 25:1 und 5:1, bevorzugt im Bereich zwischen 9,8:1 bis 6,6:1 liegen.

Für eine stabile Micellierung von Curcumin und Boswellia im erfindungsgemäßen Solubilisat hat es sich als günstig erwiesen, wenn das Verhältnis von Emulgator zur Gesamtmasse von Curcumin und Boswelliasäuren und/oder von Curcumin und Boswelliasäuren und zumindest einem ihrer Derivate im Bereich zwischen 15:1 und 3:1, bevorzugt im Bereich zwischen 10:1 und 4:1, bevorzugt im Bereich zwischen 8,8:1 bis 5,7:1 liegt.

Je nachdem, wie viel Emulgator für einen bestimmten Verwendungszweck des Solubilisats eingesetzt werden kann, bietet die Erfindung die Möglichkeit, dass das Solubilisat bis zu 20 Gew.-%, bevorzugt bis zu 15 Gew.-% Ethanol enthält. Durch Zugabe von Ethanol kann der Anteil von Polysorbat reduziert werden, was im Hinblick auf den ADI-Wert für Polysorbat einen Vorteil darstellt.

Für die Ausbildung stabiler Micellen kann es je nachdem, welche Wirkstoffe in welcher Menge solubilisiert werden sollen, hilfreich sein, dass das Solubilisat bis zu 25 Gew.-%, bevorzugt bis zu 10 Gew.-% Glycerin enthält. Auch durch Zugabe von Glycerin kann der Anteil von Polysorbat reduziert werden.

Die Solubilisate gemäß der Erfindung haben auch unter den physiologischen Bedingungen einer Magenpassage eine enge Partikelgrößenverteilung mit kleinen mittleren Partikelgrößen, bevorzugt reicht die Durchmesserverteilung der Micellen in einer Verdünnung des Solubilisats mit destilliertem Wasser im Verhältnis 1:500 bei physiologischen Bedingungen (pH 1,1 und 37°C) von etwa $d_{10}$=6 nm bis etwa $d_{90}$=16 nm. Diese Werte wurden anhand einer Volumenverteilung ermittelt. Einzelheiten zur Partikelgrößenanalyse der Micellen der Solubilisate werden unten erläutert.

Die Erfindung stellt vorteilhafterweise Solubilisate mit sehr guten entzündungshemmenden Eigenschaften zur Verfügung. Die antiinflamatorische Aktivität gemessen als Konzentration von C-reaktivem Protein (CRP) im Blutserum arthritischer Ratten liegt nach einmaliger Gabe des Solubilisats in einer Dosierung von 5 mg/kg Körpergewicht Curcumin und 10 mg/kg Körpergewicht Boswelliasäuren im Bereich von etwa 1200 pg/mL bis etwa 1500 pg/mL im Vergleich zu etwa 3200 pg/mL bis etwa 3500 pg/mL nach einer Gabe gleicher Dosierung von nativem Curcumin beziehungsweise Boswellia.

Die antiinflamatorische Wirkung eines erfindungsgemäßen Solubilisats von Curcumin und Boswellia gemessen als Konzentration von Myeloperoxidase (MPO) im Blutserum arthritischer Ratten liegt nach einmaliger Gabe des Solubilisats in einer Dosierung von 5 mg/kg Körpergewicht Curcumin und 10 mg/kg Körpergewicht Boswelliasäuren im Bereich von etwa 750 mU/mL bis etwa 815 mU/mL und damit deutlich niedriger als etwa 1150 mU/mL bis etwa 1250 mU/mL nach einer Gabe gleicher Dosierung von nativem Curcumin beziehungsweise Boswellia.

Die Enzymeinheit (U) ist eine heute mittlerweile durch das Katal abgelöste Einheit zur Angabe der Enzymaktivität. Da sich bei Verwendung des Katal die Zahlenwerte ändern, wird die Enzymeinheit (U) in Medizin und klinischer Chemie weiterhin genutzt. Eine Enzymeinheit U entspricht einem Mikro-Mol Substratumsatz pro Minute.

Einen Hinweis auf die im Vergleich zu nicht gemäß der Erfindung micellierten Zusammensetzungen von Boswellia mit Curcumin verbesserte Bioverfügbarkeit gibt die messtechnisch deutlich einfacher zugängliche Bestimmung der Trübung des Solubilisats. Die Trübung des Solubilisats ist vorzugsweise infolge der erfindungsgemäßen Formulierung kleiner als 25 FNU, bevorzugt kleiner als 3 FNU gemessen durch Streulichtmessung mit Infrarotlicht nach den Vorschriften der Norm ISO 7027 bei einer Verdünnung des Solubilisats im Verhältnis 1:500 in Wasser bei physiologischen Bedingungen (pH 1,1 und 37°C).

Im Rahmen der Erfindung kann das Solubilisat das Xanthohumol in nicht-solubilisierter, insbesondere in nativer Form, enthalten. Ein derartiges Produkt entsteht, wenn Xanthohumol-haltiges Pulver, beispielsweise ein Hopfenextrakt, zu einem oben beschriebenen Solubilisat zugegeben wird. Dadurch enthält das Solubilisat Wirkstoffe wie Boswellia und Curcumin in solubilisierter Form, das heißt, sie liegen in Micellen vor, und zusätzlich Xanthohumol, welches nicht in solubilisierter Form vorliegt, also nicht in Micellen eingeschlossen ist. Xanthohumol kann dabei als dispergierte Phase von einer Emulgatorhülle umgeben sein, beispielsweise in Form von Emulsionstropfen bzw. Partikeln einer Suspoemulsion mit dem sonstigen Solubilisat als kontinuierlicher Phase.

Um die orale Anwendung des erfindungsgemäßen Solubilisats in für den Konsumenten beziehungsweise Patienten einfacher und angenehmer Weise zu ermöglichen, stellt die Erfindung zudem eine Kapsel gefüllt mit einem oben beschriebenen oder einem entsprechenden Solubilisat von beziehungsweise mit Curcumin, Boswelliasäure und Xanthohumol zur Verfügung, wobei die Kapsel als Weichgelatinekapsel oder Hartgelatinekapsel oder als weiche, gelatinefreie Kapsel oder als harte, gelatinefreie Kapsel, beispielsweise als Cellulose-Kapsel, ausgebildet ist.

Das erfindungsgemäße Solubilisat kann im Rahmen der Erfindung zudem in andere Fluide, insbesondere Flüssigkeiten eingearbeitet werden. Dabei bleiben die wirkstoffgefüllten kleinen Micellen erhalten. Somit stellt die Erfindung auch ein Fluid enthaltend oben beschriebenes Solubilisat zur Verfügung, wobei das Fluid aus der Gruppe ausgewählt ist, welche Lebensmittel, Getränke, Kosmetika und pharmazeutische Produkte umfasst. Insbesondere kann das Fluid im Rahmen der Erfindung eine wässrige Verdünnung des Solubilisats umfassen.

Damit stellt die Erfindung auch in besonders einfacher Weise ein oben beschriebenes Solubilisats oder Fluid zur insbesondere oralen Anwendung als Nahrungsergänzungsmittel und/oder als Arzneimittel in einem Verfahren zur zur Behandlung von und/oder Vorbeugung vor mit einer Entzündung einhergehenden Krankheiten, Krebs, Alzheimer, Parkinson, Adipositas, hohen Cholesterinwerten, erhöhtem Blutzucker, metabolischem Syndrom und/oder Autoimmunkrankheiten zur Verfügung.

In einer bevorzugten Ausführungsform des erfindungsgemäßen Solubilisats ist das Solubilisat zur Anwendung als Nahrungsergänzungsmittel und/oder als Arzneimittel in einer Curcumin-Dosis im Bereich von 0,5 mg/kg Körpergewicht bis 1 mg/ kg Körpergewicht, bevorzugt mit einer Dosis von 0,81 mg/kg Körpergewicht, und in einer Boswellia-Dosis im Bereich von 1 mg/kg Körpergewicht bis 2 mg/ kg Körpergewicht, bevorzugt mit einer Dosis von 1,62 mg/kg Körpergewicht, insbesondere einmal täglich, und in einer Xanthohumol-Dosis im Bereich von 0,5 mg/kg Körpergewicht bis 1 mg/kg Körpergewicht, bevorzugt mit einer Dosis von 0,81 mg/kg Körpergewicht ausgebildet.

Zur Herstellung eines erfindungsgemäßen Solubilisats mit den Wirkstoffen Curcumin und Boswellia und Xanthohumol können entweder einzeln hergestellte Solubilisate miteinander gemischt werden oder ein Solubilisat enthaltend Curcumin und Boswellia und Xanthohumol wird direkt hergestellt.

Die Erfindung stellt des Weiteren Verfahren zum Herstellen eines oben beschriebenen Solubilisats bereit. Wird eine Co-Micellierung von Boswellia mit Curcumin und Xanthohumol angestrebt, stellt die Erfindung folgende Variante für ein Herstellungsverfahren bereit mit den Schritten

a) Vorlegen von Polysobat 80 und/oder Polysorbat 20 und/oder eine Mischung aus Polysorbat 20 und Polysorbat 80
b) Zugabe von *Boswellia serrata* Extrakt-Pulver und eines ethanolischen Auszugs der Hartharze aus Hopfen, insbesondere Xantho-Flav pur-Pulver und/oder Xantho-Flav-Pulver,
c) Zugabe von Curcumin Pulver

wobei in Schritt a) eine Erwärmung auf eine Temperatur im Bereich von 40°C bis 62°C, bevorzugt auf eine Temperatur im Bereich von 45°C bis 57°C, besonders bevorzugt auf eine Temperatur im Bereich von 48°C und 52°C, erfolgt und wobei in Schritt b) eine Erwärmung auf eine Temperatur im Bereich von 60°C bis 75°C, bevorzugt auf eine Temperatur im Bereich von 61°C bis 70°C, besonders bevorzugt auf eine Temperatur im Bereich von 63°C und 67°C erfolgt
und wobei in Schritt c) eine Erwärmung auf eine Temperatur im Bereich von 82°C bis 97°C, bevorzugt auf eine Temperatur im Bereich von 83°C bis 92°C, besonders bevorzugt auf eine Temperatur im Bereich von 85°C und 89°C erfolgt.

Durch diese Herstellungsweise wird es ermöglicht, ein Solubilisat zu produzieren, welches in wässriger Verdünnung Micellen ausbilden kann, welche sowohl mit Curcumin als auch mit Boswelliasäuren und mit Xanthohumol beladen sind. Dazu können die beiden Wirkstoffe auch bei einer entsprechend angepassten Temperaturführung in einem vorbereitenden Schritt miteinander gemischt und dann als Mischung gemeinsam zugegeben werden.

Im Rahmen der Erfindung ist es jedoch auch möglich, natives Xanthohumol in einem Solubilisat mit Curcumin und Boswelliasäuren bereitzustellen. Dafür stellt die Erfindung folgende Variante für ein Herstellungsverfahren bereit mit den Schritten

a) Vorlegen von Polysobat 80 und/oder Polysorbat 20 und/oder eine Mischung aus Polysorbat 20 und Polysorbat 80
b) Zugabe von *Boswellia serrata* Extrakt-Pulver
c) Zugabe von Curcumin Pulver

wobei in Schritt a) eine Erwärmung auf eine Temperatur im Bereich von 40°C bis 62°C, bevorzugt auf eine Temperatur im Bereich von 45°C bis 57°C, besonders bevorzugt auf eine Temperatur im Bereich von 48°C und 52°C, erfolgt und wobei in Schritt b) eine Erwärmung auf eine Temperatur im Bereich von 60°C bis 75°C, bevorzugt auf eine Temperatur im Bereich von 61°C bis 70°C, besonders bevorzugt auf eine Temperatur im Bereich von 63°C und 67°C erfolgt.
und wobei in Schritt c) eine Erwärmung auf eine Temperatur im Bereich von 82°C bis 97°C, bevorzugt auf eine Temperatur im Bereich von 83°C bis 92°C, besonders bevorzugt auf eine Temperatur im Bereich von 85°C und 89°C erfolgt,
mit einem anschließendem Schritt
d) Zugabe eines ethanolischen Auszugs der Hartharze aus Hopfen, insbesondere Xantho-Flav pur-Pulver und/oder Xantho-Flav Pulver bei einer Temperatur im Bereich von 26°C bis 30°C.

Insbesondere kann dabei vor Schritt b) ein Schritt
b1) Zugabe von Wasser bei einer Temperatur im Bereich von 40°C bis 62°C, bevorzugt bei einer Temperatur im Bereich von 45°C bis 57°C, besonders bevorzugt bei einer Temperatur im Bereich von 48°C und 52°C, durchgeführt werden.

Die Erfindung betrifft auch Solubilisate, welche sowohl allein mit Curcumin als auch allein mit Boswelliasäuren als auch allein mit Xanthohumol beladene Mizellen in wässriger Verdünnung zeigen, zumindest unmittelbar nach ihrer Herstellung. Daher stellt die Erfindung ebenso ein Verfahren zum Herstellen eines oben beschriebenen Solubilisats durch Mischen eines Curcuminsolubilisats und eines Boswellia-Solubilisats und eines Xanthohumol-Solubilisats, insbesondere im Mengenverhältnis 1:1:1, zur Verfügung.

Die Erfindung wird nachfolgend anhand von Ausführungsbeispielen näher erläutert. Dabei wurden die folgenden Komponenten verwendet.

**Boswellia**

Mit dem Begriff "Boswellia" wird im Rahmen der vorliegenden Anmeldung insbesondere ein Extrakt aus dem Harz der Weihrauchpflanze bezeichnet. Speziell wurde ein Extrakt der Art *boswellia serrata* verwendet. Dabei handelte es sich um ein durch Extraktion mittels Essigsäure-Ethylester aus dem Harz der Pflanze mit dem botanischen Namen *boswellia serrata* gewonnenes Extrakt mit dem Produktcode "HC22519" des Herstellers Frutarom Belgium N.V., Londerzeel, Belgien. Ein Solubilisat enthaltend diesen Extrakt wird wegen dessen Gehalts an Boswelliasäuren auch als "Boswelliasäuren-Solubilisat" bezeichnet.

Neben Extrakten aus dem Harz der Weihrauchpflanze können für den Zweck der erfindungsgemäßen Solubilisate auch Boswelliasäuren und/oder Derivate der Boswelliasäuren verwendet werden. Dabei kommen insbesondere die Alpha-Boswelliasäure (CAS-Nr. 471-66-9), die Beta-Boswelliasäure (CAS-Nr. 631-69-6) sowie deren Derivate 3-O-Acetyl-Alpha-Boswelliasäure (CAS-Nr. 89913-60-0), 3-O-Acetyl-Beta-Boswelliasäure (CAS-Nr. 5968-70-7), 11-Keto-Beta-Boswelliasäure (KBA, CAS-Nr. 17019-92-0) und 3-O-Acetyl-11-Keto-Beta-Boswelliasäure (AKBA, CAS-Nr. 67416-61-9) in Frage.

**Curcumin**

Als Curcumin würde das Produkt mit dem Namen "Turmeric Oleoresin Curcumin Powder 95 %" mit dem Produktcode EP-5001 der Green Leaf Extractions Pvt Limited, Kerala, Indien, verwendet. Das Curcuminpulver trägt die CAS-Nr. 458-37-7. Es handelt sich um ein Naturprodukt, welches durch Lösungsmittelextraktion der Rhizome von *Curcuma Longa* gewonnen wird. Der Curcumin-Gehalt des Pulvers beträgt nach Herstellerangaben mindestens 95 %. Dieser Curcumin-Gehalt wird durch die ASTA-Methode 18.0 bestimmt.

In den unten beschriebenen Ausführungsbeispielen können alternativ zu dem erwähnten "oleoresin turmeric 95%"-Curcuminpulver von Greenleaf als Curcumin beispielsweise auch 95%iges Curcuminextrakt von Neelam Phytoextracts, Mumbai, Indien oder Curcumin BCM-95-SG beziehungsweise Curcumin BCM-95-CG der eurochem GmbH, Gröbenzell, Deutschland oder Curcuma Oleoresin 95 % der Henry Lamotte OILS GmbH, Bremen, Deutschland verwendet werden.

**Xanthohumol**

Als Xanthohumol-Quelle wurden die Produkte "Xantho-Flav" oder "Xantho-Flav pur" der Marke "Hopsteiner" der Simon H. Steiner, Hopfen, GmbH, Mainburg, Deutschland verwendet. Bei beiden handelt es sich um ein Naturprodukt, das aus Hopfen hergestellt wird. Der aktive Inhaltsstoff ist das Hopfen-Polyphenol Xanthohumol. Es handelt sich um ein gelbfarbenes Pulver mit einem Xanthohumol-Gehalt nach Herstellerangaben zwischen 65 % und 85 % in "Xantho-Flav" und von mindestens 85 % bei "Xantho-Flav pur". Die Konzentrationen an Xanthohumol und Isoxanthohumol in "Xantho-Flav pur" werden vom Hersteller nach der UV-spektrophotometrischen Analyse oder nach HPLC EBC 7.8 über externen Kalibrierstandard reines XN (370 nm) bzw. IX (290 nm) quantifiziert. "Xantho-Flav pur" enthält das prenylierte Flavonoid Xanthohumol in sehr hoher Konzentration. Für die Ausführungsbeispiele im Rahmen der vorliegenden Anmeldung wurde "Xantho-Flav pur" der Batchnummer 9432 verwendet.

**Polysorbat 80**

Als Quelle für Polysorbat 80 wurde das Material "TEGO SMO 80 V FOOD" mit dem Spezifikationscode "K04 EU-FOOD" der Evonik Nutrition & Care GmbH, Essen, Deutschland, verwendet. Das Produkt entspricht den EU-Anforderungen des Lebensmittelzusatzstoffes E 433. In den unten beschriebenen Ausführungsbeispielen können alternativ zu dem erwähnten TEGO SMO 80 V von Evonik als Polysorbat 80 auch TEGO SMO 80 V von der InCoPA Gmbh, Illertissen, Deutschland oder Crillet 4/Tween 80-LQ-(SG) von der CRODA GmbH, Nettetal, Deutschland oder Lamesorb SMO 20 sowie Kotilen-O/1 VL von Univar oder der Kolb Distributions AG, Hedingen, Schweiz verwendet werden.

**Polysorbat 20**

Als Quelle für Polysorbat 20 wurde das Material "TEGO SML 20 V FOOD" mit dem Spezifikationscode "K09 EU-FOOD" der Evonik Nutrition & Care GmbH, Essen, Deutschland, verwendet. Das Produkt entspricht den EU-Anforderungen des Lebensmittelzusatzstoffes E 432. Alternativ zu dem erwähnten TEGO SML 20 von Evonik kann im Rahmen der Erfindung als Polysorbat 20 auch Crillet 1/Tween 20-LQ-(SG) von der CRODA GmbH, Nettetal, Deutschland verwendet werden.

Wird bei der Herstellung eines Solubilisats zugegeben, wird destilliertes Wasser verwendet.

### Ethanol

Ethanol wurde im Rahmen der vorliegenden Anmeldung von der Berkel pfälzische Spritfabrik GmbH & Co. KG bezogen. Gemäß der Spezifikation für "Neutralalkohol unvergällt" 1411U versteuert" beträgt der Gehalt an Ethanol dieses Produkts etwa 92,6 bis 95,2 Gewichts-%.

Die Partikelgrößenanalysen der Micellen in wässrigen Verdünnungen erfindungsgemäßer Solubilisate wurden gemessen nach dem Prinzip der dynamischen Lichtstreuung mit Laserlicht der Wellenlänge 780 nm. Die Partikelgrößenmessungen wurden mit dem ParticleMetrix NANOFLEX Rückstreu-Teilchenanalysator durchgeführt. Das Messprinzip beruht auf der dynamischen Lichtstreuung (DLS) in einer 180° Heterodyn-Rückstreuanordnung. Bei dieser Geometrie wird zum gestreuten Licht ein Teil des Laserstrahls dazu gemischt (Heterodyn-Technik). Wegen des geringen Lichtweges von 200 Mikrometer bis 300 Mikrometer in der Probe ist die Rückstreuung für absorbierende und hochkonzentrierte Proben von Vorteil. Die Heterodyn-Technik wirkt sich verstärkend auf das Signal/Rausch-Verhältnis und auf die Empfindlichkeit des Sub-100nm-Bereiches aus.

Das Laserlicht wird in die Y-Gabel einer Lichtfaser eingekoppelt. Zurück kommen in derselben Faser das am Saphirfenster der Probenkammer teilreflektierte Laserlicht und das von der Probe rückwärts gestreute Licht. Der Detektor im zweiten Ast der Y-Gabel nimmt die miteinander interferierenden Signale auf. Eine schnelle Fouriertransformations-Auswertung zerlegt die fluktuierenden Streulichtanteile in ein frequenzabhängiges sogenanntes "Power-Spektrum". Jeder Frequenzanteil stellt eine Brown'sche Diffusionskonstate dar und ist damit einer Partikelgröße zuzuordnen. Zur Umrechnung in eine Partikelgrößenverteilung wird die Stokes-Einstein-Formel verwendet:

$$D = k\,\frac{T}{3\pi\eta d_P}$$

In diese Gleichung gehen ein die Diffusionskonstante D, die Boltzmannkonstante k, die Temperatur T, die dynamische Viskosität $\eta$ des Mediums und der Durchmesser $d_P$ der Partikel. Ein Temperatursensor ist im Messgerät probennah in der Nähe des Saphirfensters angebracht.

Zur experimentellen Bestimmung der Trübung der erfindungsgemäßen Solubilisate werden die Trübungsmessgeräte mit einer Standardsuspension kalibriert. Die Anzeige erfolgt somit nicht in Form der gemessenen Lichtintensität, sondern als Konzentration der Kalibriersuspension. Bei der Messung einer beliebigen Suspension bedeutet also die Anzeige, dass die betreffende Flüssigkeit die gleiche Lichtstreuung verursacht wie die Standardsuspension der angezeigten Konzentration. Der international festgelegte Trübungsstandard ist Formazin. Zu den geläufigsten Einheiten gehört die Angabe "FNU", das heißt "Formazine Nephelometric Units". Dies ist die beispielsweise in der Wasseraufbereitung verwendete Einheit für die Messung bei 90° gemäß den Vorschriften der Norm ISO 7072.

Zur Herstellung eines erfindungsgemäßen Solubilisats mit den Wirkstoffen Curcumin und Boswellia können entweder einzeln hergestellte Solubilisate miteinander gemischt werden oder ein Solubilisat enthaltend Curcumin und Boswellia wird direkt hergestellt. Im Folgenden wird zunächst ein Beispiel zur Herstellung unter Verwendung zweier zuvor einzeln hergestellter Solubilisate beschrieben.

### Ausführungsbeispiel 1

Curcumin/Boswelliasäure/Xanthohumol-Solubilisat

Zunächst wird ein 7 %iges Curcumin-Solubilisat hergestellt. Dazu werden

| | |
|---|---|
| 925 g | Polysorbat 80 |
| 75 g | Curcuminpulver 95 % (= 71,2 g Curcumin) |

verwendet. Das Polysorbat 80 wird auf 48 bis 52°C erwärmt. Unter Rühren wird das Curcumin-Pulver zum Polysorbat gegeben und dabei weiter auf eine Temperatur im Bereich von 95 bis 97°C erwärmt. Die Zugabe des Pulvers erfolgt mit einer solchen Geschwindigkeit, dass es beim Rühren gleichmäßig in den Emulgator eingezogen wird. Nach Abkühlen auf eine Temperatur unterhalb von maximal 60°C wird das Curcumin-Solubilisat abgefüllt. Dieses Solubilisat wurde für die Herstellung eines Curcumin- und Boswellia-Solubilisats verwendet.

Es sei jedoch angemerkt, dass der Curcumin-Gehalt sich weiter erhöhen lässt, ohne damit negative Folgen, beispielsweise für die Stabilität der Mizellen in Kauf nehmen zu müssen. Eine Zusammensetzung aus 100 g Curcumin-Pulver 95 %ig und 900 g Polysorbat 80 führt genauso zu einem stabilen Produkt wie eine Zusammensetzung aus 120 g Curcumin-Pulver 95 %ig und 880 Polysorbat 80. Die Herstellung dieser beiden Varianten entspricht der oben beschriebenen. Neben einem 7 %igen können so bis zu 11 %ige Solubilisate hergestellt werden.

Bei einer Verdünnung im Verhältnis 1:500 in Wasser bei einem pH-Wert von 1,1 und einer Temperatur von 37°C hat das 7 %ige Curcumin-Solubilisat eine gemittelte Trübung von 0,9 FNU.

Als nächstes wurde ein 6 %iges Boswelliasäure-Solubilisat hergestellt. Dazu wurden

| | |
|---|---|
| 76 g | Boswellia serrata Extrakt 80 %ig (= 60,8 g Boswelliasäure) |
| 24 g | Wasser |
| 400 g | Polysorbat 20 |

verwendet.

Unter Erwärmung auf eine Temperatur im Bereich von 87 bis 93°C wird das Wasser mit dem Boswellia-Pulver gemischt. Unter Beibehaltung der Temperatur wird Polysorbat 20 eingearbeitet. Die Zugabe des Emulgators erfolgt dabei mit einer solchen Geschwindigkeit, dass sich die Fluide unter Rühren stabil zu einem Solubilisat homogenisieren. Bei der Herstellung kann eine starke Schaumbildung auftreten. Diese kann ignoriert werden, sofern bei der Abfüllung ein klares Solubilisat am Boden des Abnahmegefäßes zu erkennen ist.

Die Kontrolle dieser Klarheit, welche auf die vollständige Micellierung schließen lässt, erfolgt über Laserstrahlmessungen. Eine solche Laserstrahlmessung kann beispielsweise durch Beleuchten der Probe mit Hilfe eines handelsüblichen Laserpointers, insbesondere mit einer Wellenlänge im Bereich zwischen 650 nm und 1700 nm (Spektralfarbe Rot), und anschließender Sichtkontrolle des beleuchteten beziehungsweise durchleuchteten Solubilisats. Die Kontrolle wird nicht durch Probenahme und damit außerhalb des Reaktionskessels, sondern im Reaktionskessel durchgeführt. Der Laserstrahl wird durch ein Sichtglas, welches sich auf der Vorderseite des Reaktionskessels befindet, senkrecht zum Reaktionskessel gerichtet. Wenn auf der hinteren Innenseite des Reaktionskesses vollkommen frei von Streuung nur ein Lichtpunkt erscheint, sind die entstandenen Partikelstrukturen im Reaktionskessel kleiner als die Wellenlänge des sichtbaren Lichtes und somit eine optische Bestätigung, dass der Prozess der Micellierung abgeschlossen ist.

Die Abfüllung des Produktes erfolgt bei ca. 50°C.

Schließlich wird ein 10 %iges Xantho Flav-Solubilisat (≙ 9,2 % Xanthohumol) aus

| | |
|---|---|
| 100 g | Xantho Flav pur (≙ 92 g Xanthohumol) und |
| 900 g | Polysorbat 80 hergestellt. |

Dazu wird das Xantho Flav pur-Pulver durch Rühren in Polysorbat 80 eingearbeitet. Das Pulver wird dabei mit einer solchen Geschwindigkeit zugegeben, dass es gleichmäßig in den Emulgator eingezogen wird. Unter Erwärmen auf 83 bis 87°C wird weiter homogenisiert. Nachdem ein homogenes Solubilisat erreicht ist, wird auf eine Temperatur unterhalb von 60° abgekühlt. Das Xantho Flav-Solubilisat wird dann abgefüllt und dunkel und kühl, d.h. bei unter 25°C gelagert.

Die Solubilisate von Curcumin, Boswellia und Xanthohumol werden miteinander gemischt, um ein Solubilisat mit allen drei Wirkstoffen zu erhalten.

**Ausführungsbeispiel 2**

Im Rahmen der Erfindung kann anstelle des oben erwähnten Xanthohumolsolubilisats in dem Solubilisat enthaltend Curcumin, Boswellia und Xanthohumol auch das folgende Solubilisat eingesetzt werden, welches zusätzlich Ethanol enthält:

10 %iges Xantho Flav-Solubilisat (≙ 8 % Xanthohumol) mit Ethanol

Für diese Variante eines Xanthohumol-Solubilisats werden

| | |
|---|---|
| 100 g | Xantho Flav (≙ 80 g Xanthohumol), |
| 150 g | Ethanol (96 %ig) Neutralalkohol Sorte 1411U und |
| 750 g | Polysorbat 80 |

verwendet.

Zunächst wird das Xantho Flav -Pulver in Ethanol gelöst und dabei auf eine Temperatur im Bereich zwischen 48 und 52°C erwärmt. Es entsteht eine homogene Lösung. Polysorbat 80 wird dann unter Erwärmen auf 83 bis 87°C zur Lösung von Xantho Flav in Ethanol zugegeben. Die Zugabe erfolgt mit einer solchen Geschwindigkeit, dass sich die beiden Fluide durch Rühren gut homogenisieren. Das entstehende Solubilisat wird auf unter 60°C abgekühlt und abgefüllt sowie dunkel und kühl, d.h. bei Temperaturen unterhalb von 25°C gelagert.

**Ausführungsbeispiel 3**

Alternativ könnte auch ein 7 %iges Boswelliasäure-Solubilisat eingesetzt werden. Dazu werden

|  |  |
|---|---|
| 82 g | Boswellia serrata Extrakt 80 %ig |
|  | (= 65,6 g Boswelliasäure) |
| 70 g | Wasser |
| 350 g | Polysorbat 20 |
| 441 g | Polysorbat 80 |

verwendet, was einer Gesamtmenge von 943 g entspricht.

Unter Erwärmung auf eine Temperatur im Bereich von 48 bis 52°C werden Polysorbat 20 und Polysorbat 80 unter Rühren miteinander homogenisiert und dabei ineinander gelöst. Unter Beibehaltung der Temperatur wird die Emulgatormischung mit dem Wasser versetzt. Dabei wird so stark gerührt, dass das Wasser gleichmäßig in der Emulgatorlösung gelöst wird. Bei unveränderter Temperatur wird das Boswellia serrata Extrakt unter Rühren in das mit Wasser verdünnte Emulgatorgemisch eingearbeitet. Die Zugabe des Boswellia serrata Extrakts erfolgt mit einer derart langsamen Geschwindigkeit, dass dieses gleichmäßig in die verdünnte Emulgatorlösung unter Rühren eingezogen wird.

Durch Mischen mit dem oben beschriebenen Curcuminsolubilisat und einem der genannten Xanthohumolsolubilisate entsteht ein erfindungsgemäßes Solubilisat mit Curcumin, Xanthohumol und Boswellia als Wirkstoffen.

**Ausführungsbeispiel 4**

Für die Herstellung gemäß Ausführungsbeispiel 1 eines 2,9 % Curcumin-/2,5 % Boswelliasäure/3,7 % Xanthohumol-Solubilisats werden

|  |  |
|---|---|
| 500 g | 3 %iges Curcumin-Solubilisat |
| 500 g | 6 %iges Boswellia-Solubilisat und |
| 500 g | 10 %iges Xanthohumol-Solubilisat |

nach Ausführungsbeispiel 1 verwendet.

Die drei Solubilisate werden auf eine Temperatur im Bereich von 50 bis 60 °C optional erwärmt, um ihre Viskosität zu senken, d.h. die Fließfähigkeit zu verbessern. Die drei Solubilisate werden durch Rühren zu einem Mischsolubilisat mit Curcumin und Boswellia und Xanthohumol homogenisiert. Das Produkt wird auf eine Temperatur von maximal 60 °C abgekühlt und abgefüllt. Dieses Produkt eignet sich besonders zur Verwendung als Kapselfüllung.

**Ausführungsbeispiel 5**

3,3öCurcumin / 3,6%Boswelliasäure-Solubilisat mit 1,8 % Xanthohumol

Es werden

|  |  |
|---|---|
| 45 g | Boswellia serrata Extrakt 80 % (36 g Boswelliasäure) |
| 35 g | 95 %iges Curcumin-Pulver (33,25 g Curcumin) |
| 23 g | Xantho-Flav mit mindestens 80 % Xanthohumol (18,4 g Xanthohumol) |
| 60 g | Wasser |
| 50 g | Ethanol (96 %ig) Neutralalkohol Sorte 1411U |
| 350 g | Polysorbat 20 |

(fortgesetzt)

| 437 g | Polysorbat 80 |

verwendet.

Unter Erwärmung auf eine Temperatur im Bereich von 48 bis 52°C werden Polysorbat 20 und Polysorbat 80 unter Rühren miteinander homogenisiert und dabei ineinander gelöst. Unter Beibehaltung der Temperatur wird die Emulgatormischung mit dem Wasser und Ethanol versetzt. Dabei wird so stark gerührt, dass das Wasser und das Ethanol gleichmäßig in der Emulgatorlösung gelöst werden. Bei unveränderter Temperatur werden das Boswellia serrata Extrakt und das Xanthohumolpulver unter Rühren in das mit Wasser und Ethanol verdünnte Emulgatorgemisch eingearbeitet. Die Zugabe des Boswellia serrata Extrakts und des Hopfenextraktes erfolgt mit einer derart langsamen Geschwindigkeit, dass diese gleichmäßig in die verdünnte Emulgatorlösung unter Rühren eingezogen werden. Anschließend wird die Temperatur unter starkem Rühren auf einen Bereich zwischen 63°C bis 67°C erhöht. Dann wird das Curcuminpulver unter Rühren eingearbeitet. Die Temperatur wird weiter auf einen Wert im Bereich zwischen 85°C und 89°C erhöht. Dabei wird so stark gerührt, dass das Curcumin gleichmäßig in der Vorlage verteilt und homogenisiert wird.

Es erfolgt eine Abkühlung auf eine Temperatur von kleiner oder gleich 45°C.

Das dunkelgelbe, viskose Präparat mit einem Solubilisat von Curcumin und Boswelliasäure und Xanthohumol wird dann abgefüllt und dunkel und kühl, d.h. bei unter 25°C gelagert.

Für eine Partikelgrößenanalyse dieses unter Ausführungsbeispiel 5 beschriebenen Solubilisats wurde dieses Solubilisat dieses zunächst im Verhältnis 1:500 mit destilliertem Wasser verdünnt und unter ständigem Rühren mit einem Magnetrührer und mit Hilfe einer Heizplatte auf 37°C gebracht. Anschließend wurde der pH-Wert mit 32 %iger Salzsäure auf 1,1 eingestellt. Die Proben wurden im Anschluss sofort vermessen. Die Ergebnisse sind in der folgenden Tabelle zusammengestellt. Dabei wurden die Daten zweier Messungen gemittelt.

|  | $d_{10}$ (nm) | $d_{50}$ (nm) | $d_{90}$ (nm) | $d_{99}$ (nm) |
|---|---|---|---|---|
| Intensitätsverteilung | 10,18 | 15,70 | 533,0 | 3080 |
| Volumenverteilung | 7,90 | 10,96 | 15,21 | 20,37 |

Eine Trübungsmessung ergab nach einer Verdünnung in Wasser im Verhältnis 1:500 bei pH 1,1 und einer Temperatur von 37°C einen Wert von 1,9 FNU.

Das folgende Ausführungsbeispiel 6 illustriert die direkte Herstellung eines Solubilisats von Curcumin und Boswellia, welches gemäß einer weiteren Ausführungsform der Erfindung Xanthohumol in nicht-solubilisierter Form enthält.

**Ausführungsbeispiel 6**

3,3öCurcumin / 3,6%Boswelliasäure-Solubilisat mit 1,8 % Xanthohumol

Es werden

| 45 g | Boswellia serrata Extrakt 80 % (36 g Boswelliasäure) |
| 35 g | 95 %iges Curcumin-Pulver (33,25 g Curcumin) |
| 23 g | Xantho-Flav mit mindestens 80 % Xanthohumol (18,4 g Xanthohumol) |
| 60 g | Wasser |
| 50 g | Ethanol (96 %ig) Neutralalkohol Sorte 1411U |
| 350 g | Polysorbat 20 |
| 437 g | Polysorbat 80 |

verwendet.

Unter Erwärmung auf eine Temperatur im Bereich von 48 bis 52°C werden Polysorbat 20 und Polysorbat 80 unter Rühren miteinander homogenisiert und dabei ineinander gelöst. Unter Beibehaltung der Temperatur wird die Emulgatormischung mit dem Wasser und Ethanol versetzt. Dabei wird so stark gerührt, dass das Wasser und das Ethanol gleichmäßig in der Emulgatorlösung gelöst werden. Bei unveränderter Temperatur wird das Boswellia serrata Extrakt unter Rühren in das mit Wasser verdünnte Emulgatorgemisch eingearbeitet. Die Zugabe des Boswellia serrata Extrakts erfolgt mit einer derart langsamen Geschwindigkeit, dass dieses gleichmäßig in die verdünnte Emulgatorlösung unter Rühren eingezogen wird. Anschließend wir die Temperatur unter starkem Rühren auf einen Bereich zwischen 63°C bis 67°C erhöht. Das Curcuminpulver wird unter Rühren eingearbeitet. Die Temperatur wird weiter auf einen Wert im Bereich zwischen 85°C und 89°C erhöht. Dabei wird so stark gerührt, dass das Curcumin gleichmäßig in der Vorlage verteilt und homogenisiert wird.

Es erfolgt eine Abkühlung auf eine Temperatur von kleiner oder gleich 30°C. Dann wird Xantho-Flav-Pulver unter Rühren eingearbeitet. Die Zugabe des nativen Xanthohumols erfolgt mit einer derart langsamen Geschwindigkeit, dass dieses gleichmäßig unter Rühren in die Vorlage eingezogen wird. Die Temperatur wird dabei in einem Bereich zwischen 26°C und 30°C gehalten.

Auf dieser Weise liegt Xanthohumol im Gegensatz zu Curcumin und Boswelliasäure in diesem Ausführungsbeispiel nicht micelliert, sondern ausschließlich in nativer Form vor, weil sich bei niedrigen Temperaturen aus pulvrigen Rohstoffen keine Micellen bilden können. Dies wird anhand von entsprechenden Partikelmessungen (zwei deutlich unterschiedliche Fraktionen) gezeigt bzw. bestätigt.

Das dunkelgelbe, viskose Präparat mit einem Solubilisat von Curcumin und Boswelliasäure mit nativem Xanthohumol wird dann abgefüllt und dunkel und kühl, d.h. bei unter 25°C gelagert.

In gleicher Weise wie in Ausführungsbeispiel 6 kann auch das folgende SOlubilisat von Curcumin und Boswelliasäure als Basis für die Zugabe von Xanthohumol in nativer Form genutzt werden. Durch Einstellung der zugegebenen Menge an Xanthohumol ergeben sich dann die Gewichtsanteile von Curcumin und Boswelliasäuren sowie von Xanthohumol im fertigen Produkt.

**Ausführungsbeispiel 7**

5,4 % Curcumin-/6,6 % Boswelliasäure-Solubilisat als Basis für ein Produkt mit nativem Xanthohumol

Auch diese weitere Ausführungsform des erfindungsgemäßen Solubilisats mit nativem Xanthohumol wurde direkt hergestellt. Ebenfalls wie bei dem zuvor beschriebenen Curcumin-Boswelliasäure-Solubilisat erfolgte auch hier eine Co-Mizellierung der Wirkstoffe. Dazu wurden hier

| | |
|---|---|
| 82 g | Boswellia serrata Extrakt 80 %ig (= 65,6 g Boswelliasäure) |
| 57 g | Curcumin-Pulver 95 %ig (= 54,1 g Curcumin) |
| 70 g | Wasser |
| 350 g | Polysorbat 20 |
| 441 g | Polysorbat 80 |

verwendet.

Die Herstellung des 5,4 % Curcumin-/6,6 % Boswelliasäure - Solubilisats erfolgten ebenso wie die Herstellung des zuvor beschriebenen Curcumin-/Boswelliasäure-Solubilisats in Ausführungsbeispiel 6.

Nach einer Abkühlung auf eine Temperatur von kleiner oder gleich 30°C wird Xantho-Flav-Pulver in der gewünschten Menge unter Rühren eingearbeitet. Die Zugabe des nativen Xanthohumols erfolgt mit einer derart langsamen Geschwindigkeit, dass dieses gleichmäßig unter Rühren in die Vorlage eingezogen wird. Die Temperatur wird dabei in einem Bereich zwischen 26°C und 30°C gehalten.

Bei niedrigen Temperaturen kann sich keine Produktmicelle bilden. Xanthohumol liegt daher bei diesem Ausführungsbeispiel nur in nativer und nicht in micellierter Form vor. Gezeigt und bestätigt wird dies bei der entsprechenden Partikelmessung durch zwei deutlich unterschiedliche Fraktionen.

Das dunkelgelbe, viskose Präparat mit einem Solubilisat von Curcumin und Boswelliasäure mit nativem Xanthohumol wird dann abgefüllt und dunkel und kühl, d.h. bei unter 25°C gelagert.

Im Rahmen der Erfindung können die Gehalte an Curcumin und Boswelliaextrakt und Xanthohumol in den einzelnen Solubilisaten je nach Anwendungsfall auch deutlich höher als im gezeigten Beispiel eingestellt werden.

Werden höhere Beladungen mit Wirkstoff im Solubilisat vor oder ohne Zugabe von nativem Xanthohumol eingestellt, ist dies dadurch begrenzt, dass bei Überschreiten eines für die jeweilige Zusammensetzung individuellen Wirkstoffgehalts kein Solubilisat, sondern eine Emulsion hergestellt wird. Wird der Wirkstoffgehalt erhöht, sinken notwendigerweise die entsprechenden Anteile der anderen Komponenten (in Gew.-%).

Oberhalb einer spezifischen Grenze erhält man ein disperses System, das jedoch nicht wie die erfindungsgemäßen Solubilisate irreversible in Wasser löslich ist und die für diese Solubilisate unter physiologischen Bedingungen der Magenpassage, also bei physiologischen Bedingungen (pH 1,1 und 37°C), gemessene sehr niedrige Trübung aufweist. Dies ist im Rahmen der erfindungsgemäßen Variante der Zugabe von nativem Xanthohumol ohne dessen Solubilisierung für das Xanthohumol der Fall. Curcumin und Boswellia sind jedoch auch bei dieser Variante in Form von Micellen solubilisiert.

Bei einem zu hoch gewählten Gehalt an Curcumin beziehungsweise Boswellia und/oder gegebenenfalls Xanthohumol entstehen Dispersionen. Diese können (Nano-)Emulsionen sein, sie sind aber in Bezug auf den betreffenden Wirkstoff oder die betreffenden Wirkstoffe keine Solubilisate, in denen der Wirkstoff oder die Wirkstoffe in den sehr kleinen Mizellen eingeschlossen vorliegen. Nur die Solubilisate ermöglichen aber nach den Erfahrungen des Erfinders die deutlich erhöhte Bioverfügbarkeit des Wirkstoffs oder der Wirkstoffe gemäß der Erfindung, selbst wenn eine Emulsion eine höhere Wirkstoffbeladung erlaubte. Erstaunlicherweise hat es sich aber auch als vorteilhaft erwiesen, nicht-solubilisiertes Xanthohumol gemeinsam mit Curcumin und Boswelliasäure in einem Solubilisat zu verabreichen.

Die erfindungsgemäße transparente und vollständig stabil wasserlösliche Formulierung weist, ohne Hilfsstoffe wie in Weich- und Hartgelatinekapseln, in gelatinefreien Kapseln (hart und/oder weich) und in flüssigen auf Wasser basierenden Endprodukten pH-unabhängig eine stabile Transparenz auf. Produkte mit derartiger Transparenz und Wasserlöslichkeit werden seitens der relevanten Industrie dringend für innovative Produkte als Kapselfüllung gesucht. Eine Formulierung von Curcumin mit Boswellia, also mit zumindest einer Boswelliasäuren und/oder zumindest einem Derivat einer Boswelliasäure, und Xanthohumol, die diesen Anforderungen gerecht wird, existiert bisher nach Kenntnis des Erfinders noch nicht.

Infolge der erfindungsgemäßen Formulierung in einem Solubilisat mit sehr kleinen, stabilen und magensaftresistenten Mizellen schafft die Erfindung ein Solubilisat von Curcumin mit Boswellia und Xanthohumol zur Verwendung als Nahrungsergänzungsmittel und/oder als Arzneimittel, insbesondere zur Verwendung als Nahrungsergänzungsmittel und/oder als Arzneimittel mit antiinflammatorischer Wirkung.

Es ist dem Fachmann ersichtlich, dass die Erfindung nicht auf die vorstehend beschriebenen Beispiele beschränkt ist, sondern vielmehr in vielfältiger Weise variiert werden kann. Insbesondere können die Merkmale der einzeln dargestellten Beispiele auch miteinander kombiniert oder gegeneinander ausgetauscht werden.

### Patentansprüche

1.  Solubilisat enthaltend, insbesondere bestehend aus,

    Curcumin mit einem Anteil von kleiner oder gleich 10 Gew.-%, bevorzugt kleiner oder gleich 8 Gew.-%, besonders bevorzugt 3 Gew.-% bis 7 Gew.-%,
    eine oder mehrere Boswelliasäuren und/oder ein oder mehrere Boswelliasäurederivate, welche ausgewählt sind aus der Gruppe, die "KBA" 11-keto-$\beta$-Boswelliasäure (CAS 17019-92-0), "AKBA" 3-O-Acetyl-11-keto-$\beta$-Boswelliasäure (CAS 67416-16-9), "A$\alpha$BA" 3-O-Acetyl- $\alpha$-Boswelliasäure und "A$\beta$BA" 3-O-Acetyl- $\beta$-Boswelliasäure umfasst, mit einem Anteil von insgesamt kleiner oder gleich 10 Gew.-%, bevorzugt kleiner oder gleich 8 Gew.-%, besonders bevorzugt 3 Gew.-% bis 6,6 Gew.-% und
    Xanthohumol mit einem Anteil von kleiner oder gleich 10 Gew.-%, bevorzugt kleiner oder gleich 5 Gew.-%, besonders bevorzugt 1 Gew.-% bis 3 Gew.-%, und zumindest einen Emulgator, nämlich Polysorbat 80 oder Polysorbat 20 oder einer Mischung aus Polysorbat 20 und Polysorbat 80

    **dadurch gekennzeichnet, dass**
    der Emulgatoranteil, insbesondere der Polysorbatanteil, bei mindestens 70 Gew-%, bevorzugt im Bereich zwischen 75 Gew-% und 95 Gew-%, besonders bevorzugt im Bereich zwischen 79 Gew-% und 88 Gew-% liegt.

2.  Solubilisat nach Anspruch 1,
    **dadurch gekennzeichnet, dass** das Solubilisat als Quelle für die eine oder mehreren Boswelliasäuren und/oder eine oder mehrere Boswelliasäurederivate ein durch Extraktion mittels Essigsäure-Ethylester aus dem Harz der Pflanze *Boswellia serrata* gewonnenes Extrakt enthält, wobei Boswelliasäuren in diesem Auszug in einer Konzentration von mindestens 85 Gew.-% vorliegen.

3.  Solubilisat nach einem der vorangegangenen Ansprüche, **dadurch gekennzeichnet, dass**

    das Verhältnis von Emulgator zu Boswelliasäuren und/oder zu Boswelliasäuren und zumindest einem ihrer Derivate im Bereich zwischen 20:1 und 3:1, bevorzugt im Bereich zwischen 16:1 und 4:1, bevorzugt im Bereich zwischen 14:1 bis 5:1 liegt
    und/oder dass
    das Verhältnis von Emulgator zu Curcumin im Bereich zwischen 30:1 und 3:1, bevorzugt im Bereich zwischen 25:1 und 9:1, bevorzugt im Bereich zwischen 23:1 bis 12:1 liegt
    und/oder dass
    das Verhältnis von Emulgator, insbesondere von Polysorbat 80, zu Xanthohumol im Bereich zwischen 30:1 und 3:1, bevorzugt im Bereich zwischen 25:1 und 5:1, bevorzugt im Bereich zwischen 9,8:1 bis 6,6:1 liegt
und/oder dass

das Solubilisat bis zu 20 Gew.-%, bevorzugt bis zu 15 Gew.-% Ethanol enthält
und/oder dass

die Durchmesserverteilung der Micellen in einer Verdünnung des Solubilisats mit destilliertem Wasser im Verhältnis 1:500 bei pH 1,1 und 37°C von etwa $d_{10}$=6 nm bis etwa $d_{90}$=16 nm reicht
und/oder dass

die Trübung des Solubilisats kleiner als 25 FNU, bevorzugt kleiner als 3 FNU ist gemessen durch Streulichtmessung mit Infrarotlicht nach den Vorschriften der Norm ISO 7027 bei einer Verdünnung des Solubilisats im Verhältnis 1:50 in Wasser bei physiologischen Bedingungen (pH 1,1 und 37°C).

4. Solubilisat nach einem der vorangegangenen Ansprüche, **dadurch gekennzeichnet, dass**
das Solubilisat das Xanthohumol in nicht-solubilisierter, insbesondere in nativer Form, enthält.

5. Kapsel gefüllt mit einem Solubilisat nach einem der vorangegangenen Ansprüche,
**dadurch gekennzeichnet, dass**
die Kapsel als Weichgelatinekapsel oder Hartgelatinekapsel oder als weiche, gelatinefreie Kapsel oder als harte, gelatinefreie Kapsel z.B. Cellulose-Kapsel ausgebildet ist.

6. Fluid enthaltend ein Solubilisat nach einem der Ansprüche 1 bis 4,
**dadurch gekennzeichnet, dass**
das Fluid aus der Gruppe ausgewählt ist, welche Lebensmittel, Nahrungsergänzungsmittel, Getränke, Kosmetika und pharmazeutische Produkte umfasst.

7. Fluid nach Anspruch 6,
**dadurch gekennzeichnet, dass**
das Fluid eine wässrige Verdünnung des Solubilisats umfasst.

8. Solubilisat nach einem der Ansprüche 1 bis 4 oder Fluid nach einem der Ansprüche 6 oder 7 zur insbesondere oralen Anwendung als Nahrungsergänzungsmittel und/oder als Arzneimittel in einem Verfahren zur Behandlung von und/oder Vorbeugung vor mit einer Entzündung einhergehenden Krankheiten, Krebs, Alzheimer, Parkinson, Adipositas, hohen Cholesterinwerten, erhöhtem Blutzucker, metabolischem Syndrom und/oder Autoimmunkrankheiten.

9. Solubilisat zur Anwendung gemäß Anspruch 8,

zur Anwendung als Nahrungsergänzungsmittel und/oder als Arzneimittel in einer
Curcumin-Dosis im Bereich von 0,5 mg/kg Körpergewicht bis 1 mg/ kg Körpergewicht, bevorzugt mit einer Dosis von 0,81 mg/kg Körpergewicht,
und in einer Boswellia-Dosis im Bereich von 1 mg/kg Körpergewicht bis 2 mg/kg Körpergewicht, bevorzugt mit einer Dosis von 1,62 mg/kg Körpergewicht,
und in einer Xanthohumol-Dosis im Bereich von 0,5 mg/kg Körpergewicht bis 1 mg/kg Körpergewicht, bevorzugt mit einer Dosis von 0,81 mg/kg Körpergewicht,
insbesondere einmal täglich.

10. Verfahren zum Herstellen eines Solubilisats nach einem der Ansprüche 1 bis 4,
mit folgenden Schritten

a) Vorlegen von Polysobat 80 und/oder Polysorbat 20 und/oder eine Mischung aus Polysorbat 20 und Polysorbat 80
b) Zugabe von *Boswellia serrata* Extrakt-Pulver und eines ethanolischen Auszugs der Hartharze aus Hopfen,
c) Zugabe von Curcumin Pulver

wobei

in Schritt a) eine Erwärmung auf eine Temperatur im Bereich von 40°C bis 62°C, bevorzugt auf eine Temperatur im Bereich von 45°C bis 57°C, besonders bevorzugt auf eine Temperatur im Bereich von 48°C und 52°C, erfolgt und wobei

in Schritt b) eine Erwärmung auf eine Temperatur im Bereich von 60°C bis 75°C, bevorzugt auf eine Temperatur im Bereich von 61°C bis 70°C, besonders bevorzugt auf eine Temperatur im Bereich von 63°C und 67°C erfolgt. und wobei

in Schritt c) eine Erwärmung auf eine Temperatur im Bereich von 82°C bis 97°C, bevorzugt auf eine Temperatur im Bereich von 83°C bis 92°C, besonders bevorzugt auf eine Temperatur im Bereich von 85°C und 89°C erfolgt.

**11.** Verfahren zum Herstellen eines Solubilisats nach einem der Ansprüche 1 bis 4,

mit folgenden Schritten

a) Vorlegen von Polysobat 80 und/oder Polysorbat 20 und/oder eine Mischung aus Polysorbat 20 und Polysorbat 80
b) Zugabe von *Boswellia serrata* Extrakt-Pulver
c) Zugabe von Curcumin Pulver
wobei

in Schritt a) eine Erwärmung auf eine Temperatur im Bereich von 40°C bis 62°C, bevorzugt auf eine Temperatur im Bereich von 45°C bis 57°C, besonders bevorzugt auf eine Temperatur im Bereich von 48°C und 52°C, erfolgt
und wobei
in Schritt b) eine Erwärmung auf eine Temperatur im Bereich von 60°C bis 75°C, bevorzugt auf eine Temperatur im Bereich von 61°C bis 70°C, besonders bevorzugt auf eine Temperatur im Bereich von 63°C und 67°C erfolgt.
und wobei
in Schritt c) eine Erwärmung auf eine Temperatur im Bereich von 82°C bis 97°C, bevorzugt auf eine Temperatur im Bereich von 83°C bis 92°C, besonders bevorzugt auf eine Temperatur im Bereich von 85°C und 89°C erfolgt,

mit einem anschließenden Schritt
d) Zugabe eines ethanolischen Auszugs der Hartharze aus Hopfen bei einer Temperatur im Bereich von 26°C bis 30°C.

**12.** Verfahren nach Anspruch 10 oder 11,

wobei vor Schritt b) ein Schritt
b1) Zugabe von Wasser bei einer Temperatur im Bereich von 40°C bis 62°C, bevorzugt bei einer Temperatur im Bereich von 45°C bis 57°C, besonders bevorzugt bei einer Temperatur im Bereich von 48°C und 52°C, durchgeführt wird.

**13.** Verfahren zum Herstellen eines Solubilisats nach einem der Ansprüche 1 bis 4,
durch Mischen eines Curcuminsolubilisats und eines Boswellia-Solubilisats und eines Xanthohumol-Solubilisats, insbesondere im Mengenverhältnis 1:1:1.

## Claims

**1.** A solubilizate, containing and in particular consisting of

curcumin in a content of less than or equal to 10 wt.%, preferably less than or equal to 8 wt.%, most preferably 3 wt.% to 7 wt.%;
one or more boswellic acids and/or one or more boswellic acid derivatives, in particular 11 keto-$\beta$-boswellic acid, KBA, (CAS 17019-92-0), 3-O-acetyl-11-keto-$\beta$-boswellic acid, AKBA, (CAS 67416-16-9), 3-O-acetyl-$\alpha$-boswellic acid, AaBA, and/or 3-O-acetyl-$\beta$-boswellic acid, A$\beta$BA, in a total content of less than or equal to 10 wt.%, preferably less than or equal to 8 wt.%, most preferably 3 wt.% to 6.6 wt.%; and
xanthohumol in a content of less than or equal to 10 wt.%, preferably less than or equal to 5 wt.%, most preferably 1 wt.% to 3 wt.%; and
at least one emulsifier, namely polysorbate 80 or polysorbate 20 or a mixture of polysorbate 20 and polysorbate 80;

wherein the emulsifier content, in particular the polysorbate content, is at least 70 wt.%, preferably in a range between 75 wt.% and 95 wt.%, most preferably in a range between 79 wt.% and 88 wt.%.

2. The solubilizate of claim 1,
**characterized in that**
the solubilizate contains an extract from the resin of the *boswellia serrata* plant obtained by extraction with ethyl acetate, as a source for the one or more boswellic acids and/or one or more boswellic acid derivatives, wherein this extract contains boswellic acids in a concentration of at least 85 wt.%.

3. The solubilizate as claimed in any of the preceding claims,
**characterized in that**

the ratio of emulsifier to boswellic acids and/or to boswellic acids and at least one of their derivatives is in a range between 20:1 and 3:1, preferably in a range between 16:1 and 4:1, preferably in a range between 14:1 and 5:1,
and/or **in that**
the ratio of emulsifier to curcumin is in a range between 30:1 and 3:1, preferably in a range between 25:1 and 9:1, preferably in a range between 23:1 and 12:1,
and/or **in that**
the ratio of emulsifier, in particular of polysorbate 80, to xanthohumol is in a range between 30:1 and 3:1, preferably in a range between 25:1 and 5:1, preferably in a range between 9.8:1 and 6.6:1,
and/or **in that**
the solubilizate contains up to 20 wt.%, preferably up to 15 wt.% of ethanol, and/or **in that**
a diameter distribution of the micelles in a dilution of the solubilizate with distilled water in a ratio of 1:500 at pH 1.1 and 37 °C is in a range from about $d_{10} = 6$ nm to about $d_{90} = 16$ nm,
and/or **in that**
the solubilizate exhibits a turbidity of less than 25 FNU, preferably less than 3 FNU, measured by scattered light measurement using infrared light according to the specifications of the ISO 7027 standard at a dilution of the solubilizate in a ratio of 1:50 in water under physiological conditions (pH 1.1 and 37 °C).

4. The solubilizate as claimed in any of the preceding claims,
**characterized in that**
the solubilizate contains the xanthohumol in non-solubilized form, in particular in native form.

5. A capsule filled with a solubilizate as claimed in any of the preceding claims,
**characterized in that**
the capsule is in the form of a soft gelatin capsule or a hard gelatin capsule or a soft gelatin-free capsule or a hard gelatin-free capsule, for example a cellulose capsule.

6. A fluid, containing a solubilizate as claimed in any of claims 1 to 4,
**characterized in that**
the fluid is selected from the group consisting of foods, dietary supplements, beverages, cosmetics, and pharmaceutical products.

7. The fluid of claim 6,
**characterized in that**
the fluid comprises an aqueous dilution of the solubilizate.

8. The solubilizate as claimed in any of claims 1 to 4 or the fluid as claimed in any of claims 6 or 7 for, in particular oral, use as a dietary supplement and/or as a pharmaceutical drug in a method for treating and/or preventing diseases involving inflammation, cancer, Alzheimer's, Parkinson's, obesity, high cholesterol, high blood sugar, metabolic syndrome and/or autoimmune diseases.

9. The Solubilizate for use according to claim 8 for use as a dietary supplement and/or as a pharmaceutical drug

in a curcumin dose ranging from 0.5 mg/kg body weight to 1 mg/kg body weight, preferably in a dose of 0.81 mg/kg body weight;
and in a boswellia dose ranging from 1 mg/kg body weight to 2 mg/kg body weight, preferably in a dose of 1.62 mg/kg body weight;
and in a xanthohumol dose ranging from 0.5 mg/kg body weight to 1 mg/kg body weight, preferably in a dose of 0.81 mg/kg body weight;

in particular once daily.

10. A method for producing a solubilizate according to any of claims 1 to 4, comprising the steps of

(a) providing polysorbate 80 and/or polysorbate 20 and/or a mixture of polysorbate 20 and polysorbate 80;
(b) adding *boswellia serrata* extract powder and an ethanolic extract of hard resins from hops,
(c) adding curcumin powder;

wherein step (a) comprises heating to a temperature in a range from 40 °C to 62 °C, preferably to a temperature in a range from 45 °C to 57 °C, most preferably to a temperature in a range from 48 °C to 52 °C; and
wherein step (b) comprises heating to a temperature in a range from 60 °C to 75 °C, preferably to a temperature in a range from 61 °C to 70 °C, most preferably to a temperature in a range from 63 °C to 67 °C; and
wherein step (c) comprises heating to a temperature in a range from 82 °C to 97 °C, preferably to a temperature in a range from 83 °C to 92 °C, most preferably to a temperature in a range from 85 °C to 89 °C.

11. A method for producing a solubilizate according to any of claims 1 to 4, comprising the steps of

(a) providing polysorbate 80 and/or polysorbate 20 and/or a mixture of polysorbate 20 and polysorbate 80;
(b) adding *boswellia serrata* extract powder;
(c) adding curcumin powder;

wherein step (a) comprises heating to a temperature in a range from 40 °C to 62 °C, preferably to a temperature in a range from 45 °C to 57 °C, most preferably to a temperature in a range from 48 °C to 52 °C; and
wherein step (b) comprises heating to a temperature in a range from 60 °C to 75 °C, preferably to a temperature in a range from 61 °C to 70 °C, most preferably to a temperature in a range from 63 °C to 67 °C; and
wherein step (c) comprises heating to a temperature in a range from 82 °C to 97 °C, preferably to a temperature in a range from 83 °C to 92 °C, most preferably to a temperature in a range from 85 °C to 89 °C; and comprising a subsequent step of

d) adding an ethanolic extract of hard resins from hops at a temperature in a range from 26 °C to 30 °C.

12. The method of claim 10 or 11, wherein prior to step (b), a step
(b1) is performed, comprising adding water at a temperature in a range from 40 °C to 62 °C, preferably at a temperature in a range from 45 °C to 57 °C, most preferably at a temperature in a range from 48 °C to 52 °C.

13. A method for producing a solubilizate according to any of claims 1 to 4, comprising mixing a curcumin solubilizate and a boswellia solubilizate and a xanthohumol solubilizate, in particular in a quantitative ratio of 1:1:1.

**Revendications**

1. Solubilisat contenant, en particulier composé

de curcumine dans une proportion inférieure ou égale à 10 % en poids, préférablement inférieure ou égale à 8 % en poids, de manière particulièrement préférable de 3 % en poids à 7 % en poids,
d'un ou plusieurs acides de boswellia et/ou d'un ou plusieurs dérivés d'acides de boswellia choisis dans le groupe comprenant l'acide 11-céto-β-boswellique « KBA » (CAS 17019-92-0), l'acide 3-O-acétyl-11-céto-β-boswellique « AKBA » (CAS 67416-16-9), l'acide 3-O-acétyl-α-boswellique « AaBA » et l'acide 3-O-acétyl-β-boswellique « AβBA », dans une proportion totale inférieure ou égale à 10 % en poids, préférablement inférieure ou égale à 8 % en poids, de manière particulièrement préférable de 3 % en poids à 6,6 % en poids et

de xanthohumol dans une proportion inférieure ou égale à 10 % en poids, préférablement inférieure ou égale à 5 % en poids, de manière particulièrement préférable de 1 % en poids à 3 % en poids, et d'au moins un émulsifiant, notamment le polysorbate 80 ou le polysorbate 20 ou un mélange de polysorbate 20 et de polysorbate 80,

**caractérisé en ce que**
la proportion d'émulsifiant, en particulier la proportion de polysorbate, est d'au moins 70 % en poids, préférablement comprise entre 75 % en poids et 95 % en poids, de manière particulièrement préférable comprise entre 79 % en poids et 88 % en poids.

2. Solubilisat selon la revendication l,
**caractérisé en ce que** le solubilisat contient, comme source d'un ou plusieurs acides de boswellia et/ou d'un ou plusieurs dérivés d'acides de boswellia, un extrait obtenu par extraction au moyen d'ester éthylique d'acide acétique à partir de la résine de la plante *Boswellia serrata,* les acides de boswellia étant présents dans cet extrait dans une concentration d'au moins 85 % en poids.

3. Solubilisat selon l'une des revendications précédentes, **caractérisé en ce que**

le rapport entre l'émulsifiant et les acides de boswellia et/ou les acides de boswellia et au moins un de leurs dérivés est compris entre 20:1 et 3:1, préférablement compris entre 16:1 et 4:1, préférablement compris entre 14:1 et 5:1,
et/ou **en ce que**
le rapport entre l'émulsifiant et la curcumine est compris entre 30:1 et 3:1, préférablement entre 25:1 et 9:1, préférablement entre 23:1 et 12:l,
et/ou **en ce que**
le rapport entre l'émulsifiant, en particulier le polysorbate 80, et le xanthohumol est compris entre 30:l et 3:1, préférablement compris entre 25:1 et 5:1, préférablement compris entre 9,8:1 et 6,6:1,
et/ou **en ce que**
le solubilisat contient jusqu'à 20 % en poids, préférablement jusqu'à 15 % en poids d'éthanol,
et/ou **en ce que**
la répartition des diamètres des micelles dans une dilution du solubilisat avec de l'eau distillée dans un rapport de 1:500 avec un pH de 1,1 et une température de 37°C va d'environ $d_{10}$=6 nm à environ $d_{90}$=16 nm,
et/ou **en ce que**
la turbidité du solubilisat est inférieure à 25 FNU, préférablement inférieure à 3 FNU, mesurée par diffusion de la lumière à l'aide d'une lumière infrarouge selon les prescriptions de la norme ISO 7027 pour une dilution du solubilisat dans un rapport de 1:50 dans de l'eau dans des conditions physiologiques (pH 1,1 et 37°C).

4. Solubilisat selon l'une des revendications précédentes, **caractérisé en ce que**
le solubilisat contient le xanthohumol sous forme non solubilisée, en particulier sous forme native.

5. Capsule remplie d'un solubilisat selon l'une des revendications précédentes,
**caractérisée en ce que**
la capsule est conçue comme une capsule de gélatine molle ou une capsule de gélatine dure ou comme une capsule molle, exempte de gélatine, ou comme une capsule dure, exempte de gélatine, par exemple une capsule de cellulose.

6. Fluide contenant un solubilisat selon l'une des revendications 1 à 4,
**caractérisé en ce que**
le fluide est choisi dans le groupe comprenant les produits alimentaires, les compléments alimentaires, les boissons, les produits cosmétiques et les produits pharmaceutiques.

7. Fluide selon la revendication 6,
**caractérisé en ce que**
le fluide comprend une dilution aqueuse du solubilisat.

8. Solubilisat selon l'une des revendications 1 à 4 ou fluide selon l'une des revendications 6 ou 7, destiné à une utilisation notamment par voie orale comme complément alimentaire et/ou comme médicament dans un procédé de traitement et/ou de prévention des maladies inflammatoires, du cancer, de la maladie d'Alzheimer, de la maladie de Parkinson, de l'obésité, de l'hypercholestérolémie, de l'hyperglycémie, du syndrome métabolique et/ou des maladies auto-immunes.

9.  Solubilisat destiné à une utilisation selon la revendication 8,

    à une utilisation comme complément alimentaire et/ou comme médicament
    dans une dose de curcumine comprise entre 0,5 mg/kg de poids corporel et 1 mg/kg de poids corporel, préférablement dans une dose de 0,81 mg/kg de poids corporel,
    et dans une dose de boswellia comprise entre 1 mg/kg de poids corporel et 2 mg/kg de poids corporel, préférablement dans une dose de 1,62 mg/kg de poids corporel,
    et dans une dose de xanthohumol comprise entre 0,5 mg/kg de poids corporel et 1 mg/kg de poids corporel, préférablement dans une dose de 0,81 mg/kg de poids corporel,

    en particulier une fois par jour.

10. Procédé de préparation d'un solubilisat selon l'une des revendications 1 à 4,
    comprenant les étapes suivantes :

    a) présentation de polysorbate 80 et/ou de polysorbate 20 et/ou d'un mélange de polysorbate 20 et de polysorbate 80,
    b) ajout d'une poudre d'extrait de *Boswellia serrata* et d'un extrait éthanolique de résines dures de houblon,
    c) ajout de poudre de curcumine,

    à l'étape a), un chauffage à une température comprise entre 40°C et 62°C, préférablement à une température comprise entre 45°C et 57°C, de manière particulièrement préférable à une température comprise entre 48°C et 52°C, étant effectué
    et
    à l'étape b), un chauffage à une température comprise entre 60°C et 75°C, préférablement à une température comprise entre 61°C et 70°C, de manière particulièrement préférable à une température comprise entre 63°C et 67°C, étant effectué
    et
    à l'étape c), un chauffage à une température comprise entre 82°C et 97°C, préférablement à une température comprise entre 83°C et 92°C, de manière particulièrement préférable à une température comprise entre 85°C et 89°C, étant effectué.

11. Procédé de préparation d'un solubilisat selon l'une des revendications 1 à 4,
    comprenant les étapes suivantes :

    a) présentation de polysorbate 80 et/ou de polysorbate 20 et/ou d'un mélange de polysorbate 20 et de polysorbate 80,
    b) ajout d'une poudre d'extrait de *Boswellia serrata,*
    c) ajout de poudre de curcumine

    à l'étape a), un chauffage à une température comprise entre 40°C et 62°C, préférablement à une température comprise entre 45°C et 57°C, de manière particulièrement préférable à une température comprise entre 48°C et 52°C, étant effectué,
    et
    à l'étape b), un chauffage à une température comprise entre 60°C et 75°C, préférablement à une température comprise entre 61°C et 70°C, de manière particulièrement préférable à une température comprise entre 63°C et 67°C, étant effectué,
    et
    à l'étape c), un chauffage à une température comprise entre 82°C et 97°C, préférablement à une température comprise entre 83°C et 92°C, de manière particulièrement préférable à une température comprise entre 85°C et 89°C, étant effectué,
    avec une étape consécutive

    d) ajout d'un extrait éthanolique de résines dures de houblon à une température comprise entre 26°C et 30°C.

12. Procédé selon la revendication 10 ou 11,

avant l'étape b), une étape

b1) ajout d'eau à une température comprise entre 40°C et 62°C, préférablement à une température comprise entre 45°C et 57°C, de manière particulièrement préférable à une température comprise entre 48°C et 52°C, étant effectuée.

13. Procédé de préparation d'un solubilisat selon l'une des revendications 1 à 4, en mélangeant un solubilisat de curcumine et un solubilisat de boswellia et un solubilisat de xanthohumol, en particulier dans un rapport quantitatif de 1:1:1.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20160081975 A1

**In der Beschreibung aufgeführte Nicht-Patentliteratur**

- *CHEMICAL ABSTRACTS,* 17019-92-0
- *CHEMICAL ABSTRACTS,* 67416-16-9
- *CHEMICAL ABSTRACTS,* 471-66-9
- *CHEMICAL ABSTRACTS,* 631-69-6
- *CHEMICAL ABSTRACTS,* 89913-60-0
- *CHEMICAL ABSTRACTS,* 5968-70-7
- *CHEMICAL ABSTRACTS,* 67416-61-9
- *CHEMICAL ABSTRACTS,* 458-37-7